# FASCICULE DE BREVET EUROPEEN

(11) **EP 0 949 347 B1**
(45) Date de publication et mention de la délivrance du brevet: **27.11.2002**
(21) Numéro de dépôt: 99400728.4
(22) Date de dépôt: 25.03.1999
(51) Int. Cl.: C22C 38/34

(54) **Utilisation d'aciers faiblement alliés dans des applications impliquant des propriétés anti-cokage**
Verwendung niedrig legierter Stähle, die nicht zur Koksbildung neigen
Use of steels with low content of alloying elements for applications involving non-coking properties

(30) Priorité: 31.03.1998 FR 9804088
(43) Date de publication de la demande: 13.10.1999
(73) Titulaire: INSTITUT FRANCAIS DU PETROLE, 92500 Rueil Malmaison (FR)
(72) Inventeur: Lecour, Philippe, 78130 Les Mureaux (FR); Longaygue, Xavier, 78590 Noisy Le Roi (FR); Ropital, François, 92500 Rueil Malmaison (FR)

(56) Documents cités:
- EP-A- 0 338 133
- FR-A- 627 628
- US-A- 1 456 088
- US-A- 3 455 681
- US-A- 4 141 724
- US-A- 4 790 977

## Description

La présente invention concerne l'utilisation d'aciers faiblement alliés dans des applications impliquant des propriétés anti-cokage. Ces aciers sont destinés notamment à la fabrication d'éléments d'appareillages tels que des réacteurs, des fours ou des conduites ou au recouvrement de tels appareillages utilisés notamment dans des procédés de raffinage ou de pétrochimie, ces aciers ayant une résistance au cokage améliorée.

L'invention concerne également des compositions nouvelles de tels aciers présentant une résistance au cokage améliorée.

Le dépôt carboné qui se développe dans les fours lors de la conversion des hydrocarbures est généralement appelé coke. Ce dépôt de coke est néfaste dans les unités industrielles. En effet, la formation du coke sur les parois des tubes et des réacteurs entraîne notamment une diminution des échanges thermiques, des bouchages importants et donc une augmentation des pertes de charge. Pour conserver une température de réaction constante, il peut être nécessaire d'augmenter la température des parois, ce qui risque d'entraîner un endommagement de l'alliage constitutif de ces parois. On observe aussi une diminution de la sélectivité des installations et par conséquent du rendement.

On connaît la demande JP-A-03/10 4843 qui décrit un acier réfractaire anti-cokage pour tube de four de craquage à l'éthylène. Mais cet acier comporte plus de 15 % de chrome et de nickel et au moins 0,4 % de manganèse. Cet acier est développé pour limiter la formation du coke entre 750 et 900 °C pour le craquage de l'éthylène. On connaît également le brevet US-A-5 693 155 concernant des procédés pétrochimiques utilisant des aciers inoxydables peu cokants. Ces aciers contiennent au moins 10 % de nickel et au moins 10 % de chrome. Du fait de ces teneurs en chrome et en nickel, ces aciers sont plus coûteux que ceux de la présente invention.

US-A-4 790 977 décrit un acier qui comprend en pourcentage pondéral: de 0.01 à 0.3 % C, au maximum 2% Mn, plus de 2,35% jusqu'à 4% Si, de 3% à 7% Cr, au maximum 1% Ni, au maximum 0.15% N, moins de 0,3% Al, au moins un élément formateur de carbures et nitrures choisi parmi le Nb, Ta, V, Ti et Zr dans une quantité maximale de 1,0 %, et ceci de façon suffisante pour maintenir une structure ferritique, obtenir une taille de grain fine, et bloquer les joints de grains pour améliorer la résistance au fluage.

Selon l'invention, dont l'étendue est définie par les revendications, pour obtenir une bonne résistance au cokage dans la fabrication d'éléments de fours, de réacteurs ou de conduites, on utilise un acier de composition déterminée comprenant :
- au plus 0,25 % de C,
- de 1,5 à 5 % de Si,
- de 4 à 10 % de Cr,
- de 0,5 à 2 % de Mo,
- de 0,3 à 1 % de Mn,
- au plus 0,03 % de S et
- au plus 0,03 % de P,
- le complément à 100% étant essentiellement du fer.

Les aciers utilisés dans l'invention peuvent comprendre en outre :
- au plus 0,1 % de Nb,
- au plus 0,40 % de V,
- au plus 0,10% de N,
- au plus 0,05 % d'AI et
- au plus 0,4 % de Ni.

Plus particulièrement dans l'invention, on utilisera un acier qui comprend :
- environ 0,1 % de C,
- de 1,5 à 3 % de Si,
- environ 9 % de Cr,
- environ 1 % de Mo,
- environ 0,5 % de Mn et
- au plus 0,40 % de V,
- le complément à 100% étant essentiellement du fer.

On pourra encore utiliser un acier qui comprend plus particulièrement :
- environ 0,1 % de C,
- de plus de 3 à 5 % de Si,
- environ 9 % de Cr,
- environ 1 % de Mo,
- environ 0,5 % de Mn et
- au plus 0,40 % de V,
- le complément à 100% étant essentiellement du fer.

On peut selon l'invention fabriquer de toute pièce des éléments destinés à la fabrication de fours de réacteurs ou de conduites. Ces aciers peuvent être élaborés par les méthodes classiques de fonderie et de moulage, puis mis en forme par les techniques usuelles pour fabriquer par exemple des tôles, des grilles, des tubes, des profilés, des viroles ou des plaques. Ces produits semi-finis peuvent être utilisés pour construire les parties principales des fours, des réacteurs ou des conduites, ou seulement des parties accessoires ou auxiliaires de ceux-ci.

Selon la présente invention on peut aussi utiliser ces aciers sous forme de poudres pour effectuer des revêtements des parois internes de fours, de réacteurs ou de conduites. On procède alors au recouvrement des parois internes d'un four, d'un réacteur ou d'une conduite par au moins une technique choisie parmi la co-centrifugation, la technique du « plasma », la technique PVD («Physical Vapor Deposition »), la technique CVD (« Chemical Vapor Deposition »), la technique électrolytique, la technique « overlay » et le placage.

Les appareillages ou les éléments fabriqués en utilisant les aciers de composition définie plus haut peuvent être destinées à des procédés de raffinage ou de pétrochimie se déroulant à des températures de 350 à 1100 °C, par exemple, le craquage catalytique ou thermique et la déshydrogénation.

Par exemple, pendant la réaction de reformage catalytique, qui permet d'obtenir du reformat à des températures de 450 à 650°C, une réaction secondaire provoque la formation de coke. Cette formation de coke est catalytiquement activée par la présence de nickel, de fer et/ou de leurs oxydes.

Une autre application peut être le procédé de déshydrogénation de l'isobutane qui permet d'obtenir de l'isobutène à des températures de 550 à 700 °C.

L'invention porte également sur les aciers nouveaux que l'on peut utiliser dans les applications décrites ci-dessus.

Ces aciers sont définis d'une manière générale par le fait qu'ils comprennent :
- au plus 0,25 % de C,
- de plus de 2,5 à 5 % de Si,
- de 4% à 10 % de Cr,
- de 0,5 à 2 % de Mo,
- de 0,3 à 1 % de Mn,
- au plus 0,03 % de S,
- au plus 0,03 % de P,
- le complément à 100% étant essentiellement du fer.

Ces aciers peuvent comprendre en outre :
- au plus 0,1 % de Nb,
- au plus 0,40 % de V,
- au plus 0,10 % de N,
- au plus 0,05 % d'AI et
- au plus 0,4 % de Ni.

Dans une première variante de l'invention, l'acier peut avoir la composition suivante :
- environ 0,1 % de C,
- de plus de 2,5 à 3 % de Si,
- environ 9 % de Cr,
- environ 1 % de Mo,
- environ 0,5 % de Mn,
- au plus 0,40 % de V,
- le complément à 100% étant essentiellement du fer.

Dans une autre variante de l'invention, l'acier peut avoir la composition suivante :
- environ 0,1 % de C,
- de plus de 3 à 5 % de Si,
- environ 9 % de Cr,
- environ 1 % de Mo,
- environ 0,5 % de Mn,
- au plus 0,40 % de V,
- le complément à 100% étant essentiellement du fer.

L'invention sera mieux comprise et ses avantages apparaîtront plus clairement à la lecture des exemples et des essais, nullement limitatifs, qui suivent, illustrés par les figures ci-annexées, parmi lesquelles :
- la figure 1 montre les courbes de cokage de différents aciers au cours d'une réaction de reformage catalytique : et
- la figure 2 montre des courbes de cokage pour différents aciers pour une réaction de déshydrogénation de l'isobutane.

### Compositions des aciers

La composition des aciers testés dans les exemples suivants est donnée dans le tableau 1 ci-après. Ces aciers ont une structure bainitique ou martensitique revenue.

**TABLEAU 1**

| ACIERS | C | Si | Mn | Mo | Cr | S | P | V |
|---|---|---|---|---|---|---|---|---|
| A9* | 0,10 | 0,5 | 0,6 | 1,0 | 9,2 | 0,015 | <0,04 | <0,04 |
| A5* | 0,11 | 0,5 | 0,6 | 1,1 | 5,1 | 0,045 | <0,04 | <0,04 |
| B1 | 0,10 | 1,6 | 0,6 | 1,1 | 9,1 | 0,015 | <0,04 | <0,04 |
| B1 | 0,12 | 2,7 | 0,5 | 1,1 | 9,3 | 0,010 | <0,04 | <0,04 |
| B2 | 0,10 | 3,5 | 0,6 | 1,0 | 9,2 | 0,015 | <0,04 | <0,04 |
| B21 | 0,12 | 4,4 | 0,5 | 1,1 | 9,4 | 0,010 | <0,04 | <0,04 |
| B3 | 0,11 | 5 | 0,6 | 1,1 | 9,0 | 0,015 | <0,04 | <0,04 |
| C1 | 0,11 | 1,5 | 0,6 | 1,1 | 5,0 | 0,015 | <0,04 | <0,04 |

| | | | | | | | | |
|---|---|---|---|---|---|---|---|---|
| * A9 et A5 sont des aciers standards utilisés couramment pour la fabrication de fours, de réacteurs ou d'élément de réacteurs. | | | | | | | | |

Pour les tests réalisés comme décrit dans les exemples 1 et 2, les échantillons d'acier sont préparés comme indiqué ci-après :
- Les échantillons sont découpés par électro-érosion, puis polis au papier SiC # 180, pour assurer un état de surface standard et enlever la croûte d'oxyde qui a pu se former lors du découpage.
- On effectue un dégraissage dans un bain de CCl₄, puis dans un bain d'acétone et enfin dans un bain déthanol.

### Exemple 1

Les différents alliages du tableau 1 ont été testés dans un réacteur de reformage catalytique de naphta à 600 °C, réalisée avec un rapport molaire hydrogène/ hydrocarbures de 6/1. La réaction de reformage catalytique permet d'obtenir le reformat. Une réaction secondaire est la formation de coke. Aux températures utilisées pour ce procédé, le dépôt de coke est principalement constitué de coke d'origine catalytique.

Le protocole opératoire utilisé pour la réalisation des essais est le suivant :
- Les échantillons préparés comme décrit plus haut sont suspendus au bras d'une thermobalance.
- Le réacteur tubulaire est ensuite fermé. La montée en température est réalisée sous argon.
- Le mélange réactionnel constitué de naphta, d'hydrogène et d'argon est injecté dans le réacteur.
- La microbalance permet de mesurer en continu le gain de masse sur l'échantillon.

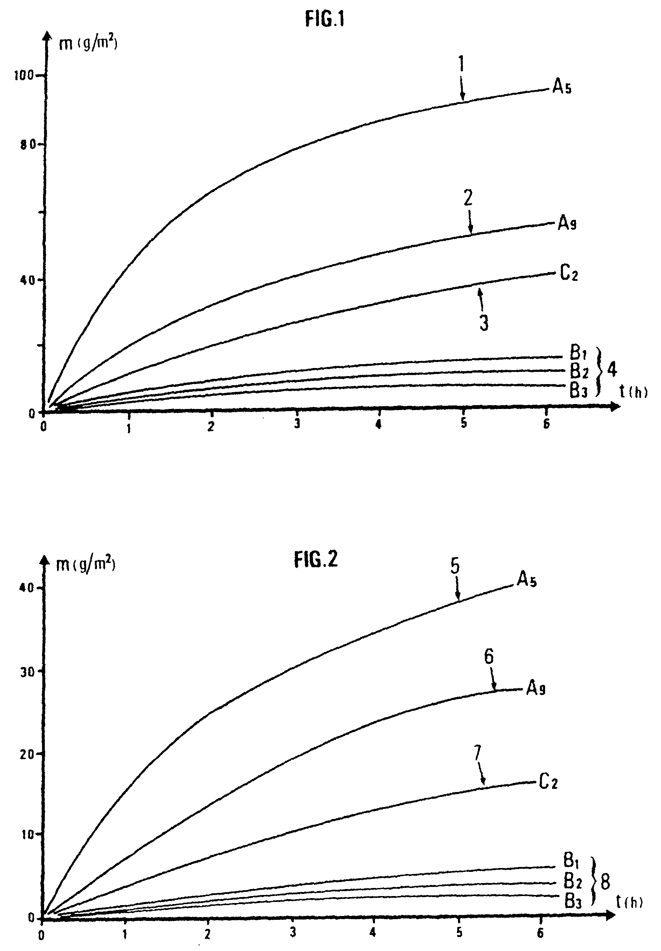

La figure 1 montre un graphique ayant en abscisses le temps en heures et en ordonnées la masse de coke qui se forme sur l'échantillon en cours de réaction, masse donnée en grammes par mètre carré (g/m²). Les courbes 1 et 2 sont relatives aux aciers A5 et A9, la courbe 3 à l'acier C1, l'ensemble des courbes 4 aux aciers B1, B2 et B3. La courbe correspondant à l'acier B11 n'a pas été représentée ; elle serait placée entre les courbes correspondant aux aciers B1 et B2. De même, la courbe correspondant à l'acier B21 n'a pas été représentée ; elle serait placée entre les courbes correspondant aux aciers B2 et B3,

Il est clair que, pour les échantillons d'aciers selon l'invention (représentés par la courbe 3 et l'ensemble des courbes 4), particulièrement pour les aciers B1, B11, B2, B21 et B3, le taux de cokage est réduit par rapport à celui observé pour les échantillons d'aciers standards A5 et A9 (courbes 1 et 2).

### Exemple 2

Un second test a été effectué dans une réaction de déshydrogénation de l'isobutane en isobutène, à une température d'environ 650 °C et avec un rapport molaire hydrogène/hydrocarbures de 3/1. Le protocole de préparation des échantillons d'acier est celui décrit plus haut et le protocole de test est le même que celui de l'exemple 1.

La figure 2 montre que le cokage des échantillons d'aciers standards A5 et A9, représenté respectivement par les courbes 5 et 6, est nettement supérieur au cokage des échantillons d'aciers B1, B2 et B3, représenté par l'ensemble des courbes 8, et à celui de l'acier C1, représenté par la courbe 7. La courbe correspondant à l'acier B11 n'a pas été représentée; elle serait placée entre les courbes correspondant aux aciers B1 et B2. De même, la courbe correspondant à l'acier B21 n'a pas été représentée ; elle serait placée entre les courbes correspondant aux aciers B2 et B3.

Pour ce second test, tous les aciers de la présente invention, qui contiennent du silicium, ont un taux de cokage inférieur à celui des aciers standards qui ne contiennent pas de proportions significatives de cet élément.

Enfin, il faut noter les bonnes caractéristiques mécaniques en température des aciers B1, B2 et B3, ainsi que celles des aciers B11 et B21, selon l'invention. Les valeurs mesurées sont quasiment les mêmes pour chacun des cinq aciers. Elles sont données dans le tableau 2 ci-dessous, dans lequel la colonne 1 correspond à la température de l'échantillon, la colonne 2 à la contrainte à la limite élastique, la colonne 3 à la contrainte à la rupture, la colonne 4 à l'allongement à la rupture et la colonne 5 correspond à la contrainte pour laquelle la rupture intervient lors du lest de fluage après 100 000 heures.

**TABLEAU 2**

| -1- T (°C) | -2- Re (MPa) | -3- Rm (MPa) | -4- A (%) | -5- tᵣᵤₚ 100 000 (MPa) |
|---|---|---|---|---|
| 500 | 180 | 410 | 40 | 350 |
| 650 | 160 | 390 | 40 | 160 |

## Revendications

1. Utilisation d'un acier comprenant :
- au plus 0,25 % de C,
- de 1,5 à 5 % de Si,
- de 4 à 10 % de Cr,
- de 0,5 à 2 % de Mo,
- de 0,3 à 1 % de Mn,
- au plus 0,03 % de S et
- au plus 0,03 % de P,
éventuellement en outre :
- au plus 0,1 % de Nb,
- au plus 0,40 % de V,
- au plus 0,10 % de N,
- au plus 0,05 % d'Al et
- au plus 0,4 % de Ni,
- le complément à 100% étant du fer et les impuretés inévitables dans la fabrication d'éléments de fours, de réacteurs ou de conduites.

2. Utilisation selon la revendication 1 dans laquelle l'acier comprend :
- environ 0,1 % de C,
- de 1,5 à 3 % de Si,
- environ 9 % de Cr,
- environ 1 % de Mo,
- environ 0,5 % de Mn et
- au plus 0,40 % de V,
- le complément à 100% étant essentiellement du fer.

3. Utilisation selon la revendication 1 dans laquelle l'acier comprend :
- environ 0,1 % de C,
- de plus de 3 à 5 % de Si,
- environ 9 % de Cr,
- environ 1 % de Mo,
- environ 0,5 % de Mn et
- au plus 0,40 % de V,
- le complément à 100% étant essentiellement du fer.

4. Utilisation selon l'une des revendications 1 à 3 dans laquelle on fabrique de toute pièce un élément entrant dans la confection de fours, de réacteurs ou de conduites.

5. Utilisation selon l'une des revendications 1 à 3 dans laquelle on procède au recouvrement des parois internes d'un four, d'un réacteur ou d'une conduite par au moins une technique choisie parmi la co-centrifugation, la technique du « plasma », la technique PVD (« Physical Vapor Deposition »), la technique CVD (« Chemical Vapor Deposition »). la technique électrolytique, la technique « overlay » et le placage.

6. Utilisation selon l'une des revendications 1 à 5 à la fabrication des fours, de réacteurs ou de conduites, en totalité ou en partie, destinés à des procédés de raffinage ou de pétrochimie se déroulant à des températures de 350 à 1100 °C.

7. Utilisation selon la revendication 6 **caractérisée en ce que** ledit procédé comporte une réaction de reformage catalytique de naphta à des températures de 450 à 650 °C.

8. Utilisation selon la revendication 6 **caractérisée en ce que** ledit procédé comporte une réaction de déshydrogénation de l'isobutane à des températures de 550 à 700 °C.

9. Acier utilisable selon l'une des revendications 1 à 8 **caractérisé en ce qu'**il comprend :
- au plus 0,25 % de C,
- de plus de 2,5 à 5 % de Si,
- de 4 à 10 % de Cr,
- de 0,5 à 2 % de Mo,
- de 0,3 à 1 % de Mn,
- au plus 0,03 % de P et
- au plus 0,03 % de P,
éventuellement en outre :
- au plus 0,1 % de Nb,
- au plus 0,40 % de V,
- au plus 0,10% de N,
- au plus 0,05 % d'Al et
- au plus 0,4 % de Ni,
- le complément à 100% étant du fer et les impuretés inévitables.

10. Acier selon la revendication 9 **caractérisé en ce qu'**il comprend :
- environ 0,1 % de C,
- de plus de 2,5 à 3 % de Si,
- environ 9 % de Cr,
- environ 1 % de Mo,
- environ 0,5 % de Mn et
- au plus 0,40 % de V,
- le complément à 100% étant essentiellement du fer.

11. Acier selon la revendication 9 **caractérisé en ce qu'**il comprend :
- environ 0,1 % de C,
- de plus de 3 à 5 % de Si,
- environ 9 % de Cr,
- environ 1 % de Mo,
- environ 0,5 % de Mn et
- au plus 0,40 % de V,
- le complément à 100% étant essentiellement du fer.

## Patentansprüche

1. Verwendung eines Stahles, welcher umfasst:
- höchstens 0,25% C,
- 1,5 bis 5% Si,
- 4 bis 10% Cr,
- 0,5 bis 2% Mo,
- 0,3 bis 1% Mn,
- höchstens 0,03% S und
- höchstens 0,03% P,
gegebenenfalls im übrigen:
- höchstens 0,1% Nb,
- höchstens 0,40% V,
- höchstens 0,10% N,
- höchstens 0,05% Al und
- höchstens 0,4% Ni,
- wobei die Vervollständigung zu 100% Eisen und die bei der Herstellung der Elemente von Öfen, Reaktoren oder Leitungen unvermeidlichen Unreinheiten sind.

2. Verwendung gemäß Anspruch 1, bei der der Stahl umfasst:
- etwa 0,1% C,
- 1,5 bis 3% Si,
- etwa 9% Cr,
- etwa 1% Mo,
- etwa 0,5% Mn und
- höchstens 0,40% V,
- wobei die Vervollständigung zu 100% im wesentlichen Eisen ist.

3. Verwendung nach Anspruch 1, bei der der Stahl umfasst:
- etwa 0,1% C,
- von mehr als 3 bis 5% Si,
- etwa 9% Cr,
- etwa 1% Mo,
- etwa 0,5% Mn und
- höchstens 0,40% V,
- wobei die Vervollständigung zu 100% im wesentlichen Eisen ist.

4. Verwendung nach einem der Ansprüche 1 bis 3, bei der man aus einem Stück ein in die Konfektionierung von Öfen, Reaktoren oder Leitungen eingehendes Element herstellt.

5. Verwendung nach einem der Ansprüche 1 bis 3, bei der man die Bedeckung der inneren Wände eines Ofens, eines Reaktors oder einer Leitung durch wenigstens eine Technik betreibt, die unter der Co-Zentrifugierung, der "Plasma"-Technik, der PVD ("Physical Vapor Deposition"), der CVD-Technik ("Chemical Vapor Deposition"), der elektrolytischen Technik, der "Overlay"-Technik und dem Auskleiden gewählt ist.

6. Verwendung nach einem der Ansprüche 1 bis 5 bei der Herstellung von Öfen, Reaktoren oder Leitungen, welche vollständig oder teilweise für Verfahren zur Raffinierung oder Petrochemie vorgesehen sind, welche sich bei Temperaturen von 350 bis 1100°C abspielen.

7. Verwendung nach Anspruch 6, **dadurch gekennzeichnet, dass** das Verfahren eine katalytische Naphtareformierungsreaktion bei Temperaturen von 450 bis 650°C umfasst.

8. Verwendung nach Anspruch 6, **dadurch gekennzeichnet, dass** das Verfahren eine Dehydrierungsreaktion von Isobutan bei Temperaturen von 550 bis 700°C umfasst.

9. Stahl, verwendbar nach einem der Ansprüche 1 bis 8, **dadurch gekennzeichnet, dass** er umfasst:
- höchstens 0,25% C,
- von mehr als 2,5 bis 5% Si,
- 4 bis 10% Cr,
- 0,5 bis 2% Mo,
- 0,3 bis 1% Mn,
- höchstens 0,03% S und
- höchstens 0,03% P,
gegebenenfalls im übrigen:
- höchstens 0,1% Nb,
- höchstens 0,40% V,
- höchstens 0,10% N,
- höchstens 0,05% Al und
- höchstens 0,4% Ni,
- wobei die Vervollständigung zu 100% Eisen und die unvermeidlichen Unreinheiten sind.

10. Stahl nach Anspruch 9, **dadurch gekennzeichnet, dass** er umfasst:
- etwa 0,1% C,
- von mehr als 2,5 bis 3% Si,
- etwa 9% Cr,
- etwa 1% Mo,
- etwa 0,5% Mn und
- höchstens 0,40% V,
- wobei die Vervollständigung zu 100% im wesentlichen Eisen ist.

11. Stahl nach Anspruch 9, **dadurch gekennzeichnet, dass** er umfasst:
- etwa 0,1% C,
- von mehr als 3 bis 5% Si,
- etwa 9% Cr,
- etwa 1% Mo,
- etwa 0,5% Mn und
- höchstens 0,40% V,
- wobei die Vervollständigung zu 100% im wesentlichen Eisen ist.

## Claims

1. Use of a steel comprising:
• at most 0.25% of C;
• 1.5% to 5% of Si;
• 4% to 10% of Cr;
• 0.5% to 2% of Mo;
• 0.3% to 1% of Mn;
• at most 0.03% of S; and
• at most 0.03% of P;
and optionally
• at most 0.1% of Nb;
• at most 0.40% of V;
• at most 0.10% of N;
• at most 0.05% of Al; and
• at most 0.4% of Ni,
• the complement to 100% being iron, and the unavoidable impurities for the production of furnace, reactor or conduit elements.

2. Use according to claim 1, in which the steel comprises:
• about 0.1% of C;
• 1.5% to 3% of Si;
• about 9% of Cr;
• about 1% of Mo;
• about 0.5% of Mn; and
• at most 0.40% of V;
• the complement to 100% being essentially iron.

3. Use according to claim 1, in which the steel comprises:
• about 0.1% of C;
• more than 3% to 5% of Si;
• about 9% of Cr;
• about 1% of Mo;
• about 0.5% of Mn; and
• at most 0.40% of V;
• the complement to 100% being essentially iron.

4. Use according to any one of claims 1 to 3, in which elements of furnaces, reactors or conduits are produced in one piece.

5. Use according to any one of claims 1 to 3, in which the internal walls of a furnace, reactor or conduit are coated using at least one technique selected from co-centrifugation, the plasma technique, PVD (Physical Vapour Deposition), CVD (Chemical Vapour Deposition), an electrolytic technique, an overlay technique and plating.

6. Use according to any one of claims 1 to 5 for the production of all or a portion of a furnace, reactor or conduit intended for refining or petrochemical processes carried out at temperatures of 350°C to 1100°C.

7. Use according to claim 6, **characterized in that** said process comprises a catalytic naphtha reforming reaction carried out at temperatures of 450°C to 650°C.

8. Use according to claim 6, **characterized in that** said process comprises isobutane dehydrogenation carried out at temperatures of 550°C to 700°C.

9. A steel for use in accordance with any one of claims 1 to 8, **characterized in that** it comprises:
• at most 0.25% of C;
• more than 2.5% and up to 5% of Si;
• 4% to 10% of Cr;
• 0.5% to 2% of Mo;
• 0.3% to 1% of Mn;
• at most 0.030% of S; and
• at most 0.03% of P;
and optionally
• at most 0.1% of Nb;
• at most 0.40% of V;
• at most 0.10% of N;
• at most 0.05% of Al; and
• at most 0.4% of Ni,
• the complement to 100% being iron, and the unavoidable impurities.

10. A steel according to claim 9, **characterized in that** it comprises:
• about 0.1% of C;
• 2.5% to 3% of Si;
• about 9% of Cr;
• about 1% of Mo;
• about 0.5% of Mn; and
• at most 0.40% of V;
• the complement to 100% being essentially iron.

11. A steel according to claim 9, **characterized in that** it comprises:
• about 0.1% of C;
• more than 3% to 5% of Si;
• about 9% of Cr;
• about 1% of Mo;
• about 0.5% of Mn; and
• at most 0.40% of V;
• the complement to 100% being essentially iron.
